# EUROPÄISCHE PATENTANMELDUNG

(11) **EP 1 964 545 A1**
(43) Veröffentlichungstag der Anmeldung: **03.09.2008**
(21) Anmeldenummer: 08103575.0
(22) Anmeldetag: 23.10.2006
(51) Int. Cl.: A61K 8/34, A61K 8/49, A61Q 17/04

(54) **Kosmetische Zubereitung mit 1,2-Alkandiol(e) und Triazin(en)**

(30) Priorität: 25.10.2005 DE 102005051858
(62) Teilanmeldung aus: 06122723.7
(71) Anmelder: Beiersdorf AG, 20245 Hamburg (DE)
(72) Erfinder: Behrens, Svea, 21109, Hamburg (DE); Bleckmann, Andreas, 22926, Ahrensburg (DE); Clausen, Andreas, 20146, Hamburg (DE); Meiring, Uta, 21077, Hamburg (DE); Nielsen, Jens, 25448, Henstedt-Ulzburg (DE)

(57) **Zusammenfassung**

Kosmetische Zubereitung enthaltend ein oder mehrere 1,2-Alkandiole sowie ein oder mehrere UV-Lichtschutzfilter aus der Gruppe der Triazinderivate.

## Beschreibung

Die vorliegende Erfindung betrifft eine kosmetische Zubereitung enthaltend ein oder mehrere 1,2-Alkandiole sowie ein oder mehrere UV-Lichtschutzfilter aus der Gruppe der Triazinderivate.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Eine wichtige Gruppe von UV-Lichtschutzfiltern baut auf dem Strukturelement des Triazins auf.

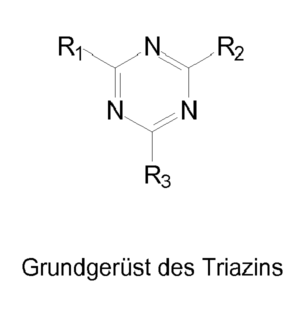

UV-Lichtschutzfilter, die das Grundgerüst des Triazins aufweisen (Triazinderivate) haben den Nachteil, dass sie in kosmetischen Zubereitungen nur schwer löslich sind. Dieser Sachverhalt führt insbesondere dann zu Problemen, wenn Zubereitungen mit einem hohen Lichtschutzfaktor hergestellt werden sollen, da in diesen Fällen nur ungenügende Mengen an Triazinderivaten in die Zubereitung eingearbeitet werden können.

Es war daher die Aufgabe der vorliegenden Erfindung Zubereitungen zu entwickeln, in die größere Mengen an Triazinderivaten eingearbeitet werden können. Insbesondere war es die Aufgabe der vorliegenden Erfindung, Zubereitungen mit einem hohen Lichtschutzfilterfaktor zu entwickeln. Diese Zubereitungen sollten, aufgrund des auf die Schutzbedürfnisse der menschlichen Haut angepassten Absorptionsspektrums von Triazinderivaten, einen besonders hohen Anteil an dieser UV-Filterklasse aufweisen.

Überraschend gelöst werden die Aufgaben durch eine kosmetische Zubereitung enthaltend
a) ein oder mehrere 1,2-Alkandiole und
b) ein oder mehrere UV-Lichtschutzfilter aus der Gruppe der Triazinderivate.

Außerdem werden die Aufgaben überraschend gelöst durch die Verwendung von 1,2-Alkandiolen zur Erhöhung der Löslichkeit von UV-Lichtschutzfiltern aus der Gruppe der Triazinderivate.

Zwar beschreiben die EP 1 238 651 und die EP 1 078 638 Zubereitungen mit Lichtschutzfiltern und Alkandiolen, doch konnten diese Schriften nicht den Weg zur vorliegenden Erfindung weisen.

Es ist erfindungsgemäß von Vorteil, wenn die erfindungsgemäße Zubereitung ein oder mehrere 1,2-Alkandiole in einer Gesamtkonzentration von 0,3 bis 3,0 Gew.-%, bezogen auf das Gesamtgewicht der Zubereitung, enthält.

Es ist erfindungsgemäß bevorzugt, wenn die erfindungsgemäße Zubereitung ein oder mehrere 1,2-Alkandiole in einer Gesamtkonzentration von 0,5 bis 2,0 Gew.-%, bezogen auf das Gesamtgewicht der Zubereitung, enthält.

Der Begriff "erfindungsgemäß" bezieht sich im Rahmen dieser Offenbarung sowohl auf die erfindungsgemäße Zubereitung als auch auf die erfindungsgemäße Verwendung. Entsprechend gilt der Begriff "erfindungsgemäße Zubereitung" auch für die erfindungsgemäßen Verwendungen.

Es ist vorteilhaft im Sinne der vorliegenden Erfindung, wenn die Zubereitung ein oder mehrere UV-Lichtschutzfilter aus der Gruppe der Triazinderivate in einer Gesamtkonzentration von 0,3 bis 3,0 Gew.-%, bezogen auf das Gesamtgewicht der Zubereitung, enthält.

Es ist bevorzugt im Sinne der vorliegenden Erfindung, wenn die Zubereitung ein oder mehrere UV-Lichtschutzfilter aus der Gruppe der Triazinderivate in einer Gesamtkonzentration von 0,5 bis 2,0 Gew.-%, bezogen auf das Gesamtgewicht der Zubereitung, enthält.

Vorteilhafte Ausführungsformen der vorliegenden Erfindung sind dadurch gekennzeichnet, dass das Gewichtsverhältnis der Gesamtmenge an 1,2-Alkandiolen zur Gesamtmenge an UV-Lichtschutzfiltern aus der Gruppe der Triazinderivate in der Zubereitung von 2:1 bis 1:2, besonders bevorzugt 1:1 beträgt.

Es ist besonders bevorzugt im Sinne der vorliegenden Erfindung, wenn als 1,2-Alkandiol 1,2-Hexandiol eingesetzt wird.

In erfindungsgemäß bevorzugten Ausführungsformen der vorliegenden Erfindung werden als UV-Lichtschutzfilter aus der Gruppe der Triazinderivate ein oder mehrere Verbindungen aus der Gruppe der folgenden Verbindungen gewählt:
- 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb S),
- Dioctylbutylamidotriazon (INCI: Dioctylbutamidotriazone),
- 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A),
- 4,4',4"-(1,3,5-Triazin-2,4,6-triyitriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird)
- 2,4,6-Tribiphenyl-4-yl-1,3,5-triazin.

In erfindungsgemäß besonders bevorzugten Ausführungsformen der vorliegenden Erfindung werden als UV-Lichtschutzfilter aus der Gruppe der Triazinderivate ein oder mehrere Verbindungen aus der Gruppe der folgenden Verbindungen gewählt:
- 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb S),
- Dioctylbutylamidotriazon (INCI: Dioctylbutamidotriazone),
- 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A),
- 2,4,6-Tribiphenyl-4-yl-1,3,5-triazin.

Darüber hinaus kann die erfindungsgemäße Zubereitung vorteilhaft weitere UV-Lichtschutzfilter enthalten. Vorteilhaft sind dabei insbesondere die nach der Kosmetikverordnung zugelassenen UV-Lichtschutzfilter. Derartige zusätzliche Filter können erfindungsgemäß vorteilhaft in Konzentrationen von 0,01 bis 30 Gew.-%, bezogen auf das Gesamtgewicht der Zubereitung in dieser enthalten sein. Dabei ist es erfindungsgemäß bevorzugt, wenn ein oder mehrere weitere UV-Lichtschutzfilter gewählt werden aus der Gruppe Phenylen-1,4-bis-(2-benzimidazyl)-3,3'-5,5'-tetrasulfonsäuresalze, 2-Phenylbenzimidazol-5-sulfonsäuresalze,1,4-di(2-oxo-10-Sulfo-3-bornylidenmethyl)-Benzol und dessen Salze, 4-(2-Oxo-3-bornylidenmethyl)benzolsulfonsäuresalze, 2-Methyl-5-(2-oxo-3-bornylidenmethyl)sulfonsäuresalze, 2,2'-Methylen-bis-(6-(2H-benzotriazol-2-yl)-4-(1,1,3,3-tetramethylbutyl)-phenol), 2-(2H-benzotriazol-2-yl)-4-methyl-6-[2-methyl-3-[1,3,3,3-tetramethyl-1-[(trimethylsilyl)oxy]disiloxanyl]propyl]-phenol, 3-(4-Methylbenzyliden)campher, 3-Benzylidencampher; 4-(Dimethylamino)-benzoesäure(2-ethylhexyl)ester, 4-(Dimethylamino)benzoesäure-amylester; 4-Methoxybenzalmalonsäuredi(2-ethylhexyl)ester; Dioctylbutylamidotriazon, 4-Methoxyzimtsäure(2-ethylhexyl)ester, 4-Methoxyzimtsäureisopentylester; 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydoxybenzoyl)-benzoesäurehexylester 4-(tert.-Butyl)-4'-methoxydibenzoylmethan, Homomenthylsalicylat, 2-Ethylhexyl-2-hydroxybenzoat, 2-Ethylhexyl-2-cyano-3,3-diphenylacrylat, 3-(4-(2,2-bis Ethoxycarbonylvinyl)-phenoxy)propenyl)-methoxysiloxan / Dimethylsiloxan - Copolymer, Titandioxid, Zinkoxid.

Erfindungsgemäß bevorzugt ist die erfindungsgemäße Zubereitung frei von 3-(4-Methylbenzyliden)campher.

Erfindungsgemäße kosmetische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe, einen Schaum oder auch ein Aerosol darstellen.

Dabei ist es erfindungsgemäß bevorzugt, wenn die kosmetische Zubereitung in Form einer Emulsion und besonders bevorzugt, wenn die Zubereitung in Form einer O/W-Emulsion vorliegt. Erfindungsgemäß bevorzugt sind derartige Emulsionen frei von Natriumdihydroxycetylphosphat (Sodium Dihydroxycetyl Phosphate, z. B. Dragophos S).

Es ist erfindungsgemäß vorteilhaft, wenn die erfindungsgemäße Zubereitung Tocopherylacetat enthält. Tocopherylacetat ist erfindungsgemäß vorteilhaft in einer Konzentration von 0,1 bis 1,0 Gew.-% und bevorzugt in einer Konzentration von 0,3 bis 0,7 Gew.-%, bezogen auf das Gesamtgewicht der Zubereitung, in der Emulsion enthalten.

Die erfindungsgemäße Zubereitung kann erfindungsgemäß vorteilhaft als Salbe, Creme oder Lotion (ggf. sprühbar) vorliegen. Die erfindungsgemäße Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- odermonobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner (z. B. Dihydroxyaceton), Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, jeweils einzeln oder in Kombination.

Die Ölphase der erfindungsgemäßen Zubereitung kann alle herkömmlichen Bestandteile von in der Kosmetik eingesetzten Öl-, Fett- und Wachskomponenten enthalten.

Die erfindungsgemäße Zubereitung kann erfindungsgemäß vorteilhaft kosmetische Wirk- und Pflegestoffe enthalten, beispielsweise die nach der Kosmetikverordnung zugelassenen Konservierungsmittel oder Konservierungshelfer. Als weitere Pflegestoffe können insbesondere Niacinamid, Panthenol, Aloe vera, Hammamelis-Extrakt, Polidocanol, Vitamin E, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gew.-%, bezogen auf das Gesamtgewicht der Zubereitung eingesetzt werden. Derartige Wirkstoffe können erfindungsgemäß vorteilhaft in Konzentrationen von 0,01 bis 30 Gew.-%, bezogen auf das Gesamtgewicht der Zubereitung in dieser enthalten sein.

Die erfindungsgemäßen Zubereitungen sind erfindungsgemäß bevorzugt frei von lodopropynylbutylcarbamat.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gew.-%, bezogen auf das Gesamtgewicht der Zubereitung eingesetzt.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

### Beispiele (O/W-Emulsionen)

| **Beispiel** | **1** | **2** | **3** | **4** | **5** | **6** | **7** | **8** |
|---|---|---|---|---|---|---|---|---|
| Glyceryl Stearate Citrat | | 1,0 | | 2,0 | | | | 2,0 |
| PEG-40 Stearat | 1,0 | | | | | 1,5 | | |
| Polyglycerylmethylglucose-distearat | | | 2,0 | | 3,0 | | 1,5 | 0,5 |
| Glycerylstearate | 1,5 | | | | | 1,5 | | |
| Cetyl Alkohol | | 0,5 | | 2,0 | 1,5 | 0,75 | 1,0 | |
| Stearyl Alcohol | | 0,5 | 0,5 | | | 0,75 | | |
| Cetearyl Alkohol | 2,0 | | | | | | | 1,5 |
| Caprylic/Capric Triglycerid | 5,0 | 4,0 | | 5,0 | | 6,0 | 3,0 | 0,5 |
| Ethylhexylcocoat | | | 2,0 | | | | 1,0 | |
| Octyldodecanol | | 1,0 | 3,0 | | 5,0 | | 2,0 | |
| Mineral Oil | | | 2,0 | | | | | |
| Hydriertes Polyisobuten | 1,0 | | | | | | | |
| Polydecen | | | | 2,0 | | | | |
| Cyclomethicon | 2,0 | | | | | 3,0 | | |
| Dimethicon | 1,0 | | | | | | | 1,5 |
| Phenyltrimethicon | | | | | | 1,0 | | |
| Dicaprylyl Carbonat | | 2,0 | | | 2,0 | | | 3,5 |
| Natürliche Öle (wie z. B. Jojobaöl / Sonnenblumenöl) | 1,5 | | 3,0 | 0,5 | 1,0 | | 2,0 | 2,5 |

| **1,2 Hexandiol** | **0,5** | **0,75** | **3,0** | **0,3** | **2,0** | **1,5** | **1,0** | **0,75** |
|---|---|---|---|---|---|---|---|---|
| Aniso Triazin | 0,5 | 0,25 | 1,5 | 0,3 | | 2,0 | 0,75 | 0,5 |
| Octyl Triazone | 0,5 | 0,5 | 1,5 | 0,2 | 3,0 | | 0,5 | 1,0 |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,7 | | | 0,4 |
| Parabene | 0,4 | | 0,3 | | 0,3 | | 0,2 | |
| Hexamidin Diisethionat | | 0,1 | | | 0,05 | | 0,1 | |
| Imidodiazolydynl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| Glycerin | 10,0 | 3,0 | 7,0 | 8,0 | 15,0 | 20,0 | 0,5 | 2,0 |
| Tocopherylacetat | 0,2 | 0,5 | 0,75 | | | 0,3 | | 1,0 |
| Alcohol Denat. | 5,0 | | 2,5 | | | 7,5 | | 7,5 |
| Wasser | ad.10 0 | ad100 | ad100 | ad100 | ad100 | ad100 | ad100 | ad100 |
| | | | | | | | | |

## Patentansprüche

1. Kosmetische Zubereitung enthaltend
a) ein oder mehrere 1,2-Alkandiole und
b) ein oder mehrere UV-Lichtschutzfilter aus der Gruppe der Triazinderivate,
**dadurch gekennzeichnet, dass** die Zubereitung Tocopherylacetat enthält.

2. Kosmetische Zubereitung nach Anspruch 1, **dadurch gekennzeichnet, dass** die Zubereitung ein oder mehrere 1,2-Alkandiole in einer Gesamtkonzentration von 0,3 bis 3,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung ein oder mehrere UV-Lichtschutzfilter aus der Gruppe der Triazinderivate in einer Gesamtkonzentration von 0,3 bis 3,0 Gewichts-%, bezogen auf das Gesamtgewicht der Zubereitung, enthält.

4. Kosmetische Zubereitung nach einem der vorhergehenden Ansprüche, **dadurch gekennzeichnet, dass** das Gewichtsverhältnis der Gesamtmenge an 1,2-Alkandiolen zur Gesamtmenge an UV-Lichtschutzfiltern aus der Gruppe der Triazinderivate in der Zubereitung von 2:1 bis 1:2, besonders bevorzugt 1:1 beträgt.

5. Kosmetische Zubereitung nach einem der vorhergehenden Ansprüche, **dadurch gekennzeichnet, dass** als 1,2-Alkandiol 1,2-Hexandiol eingesetzt wird.

6. Kosmetische Zubereitung nach einem der vorhergehenden Ansprüche, **dadurch gekennzeichnet, dass** als UV-Lichtschutzfilter aus der Gruppe der Triazinderivate ein oder mehrere Verbindungen aus der Gruppe 2,4-Bis-{[4-(2-ethyl-hexyloxy)-2-hydroxy]-phenyl}-6-(4-methoxyphenyl)-1,3,5-triazin, Dioctylbutylamidotriazon, 2,4-bis-[5-1 (dimethylpropyl)benzoxazol-2-yl-(4-phenyl)-imino]-6-(2-ethylhexyl)-imino-1,3,5-triazin, 4,4',4"-(1,3,5-Triazin-2,4,6-triyltriimino)-tris-benzoësäure-tris(2-ethylhexylester), 2,4,6-Tribiphenyl-4-yl-1,3,5-triazin eingesetzt werden.

7. Kosmetische Zubereitung nach einem der vorhergehenden Ansprüche, **dadurch gekennzeichnet, dass** als UV-Lichtschutzfilter aus der Gruppe der Triazinderivate ein oder mehrere Verbindungen aus der Gruppe 2,4-Bis-{[4-(2-ethyl-hexyloxy)-2-hydroxy]-phenyl}-6-(4-methoxyphenyl)-1,3,5-triazin, Dioctylbutylamidotriazon, 2,4-bis-[5-1(dimethylpropyl)benzoxazol-2-yl-(4-phenyl)-imino]-6-(2-ethylhexyl)-imino-1,3,5-triazin, 2,4,6-Tribiphenyl-4-yl-1,3,5-triazin eingesetzt werden.

8. Kosmetische Zubereitung nach einem der vorhergehenden Ansprüche, **dadurch gekennzeichnet, dass** die Zubereitung weitere UV-Lichtschutzfilter gewählt aus der Gruppe Phenylen-1,4-bis-(2-benzimidazyl)-3,3'-5,5'-tetrasulfonsäuresalze, 2-Phenylbenzimidazol-5-sulfonsäuresalze,1,4-di(2-oxo-10-Sulfo-3-bornylidenmethyl)-Benzol und dessen Salze, 4-(2-Oxo-3-bornylidenmethyl)benzolsulfonsäuresalze, 2-Methyl-5-(2-oxo-3-bornylidenmethyl)sulfonsäuresalze, 2,2'-Methylen-bis-(6-(2H-benzotriazol-2-yl)-4-(1,1,3,3-tetramethylbutyl)-phenol), 2-(2H-benzotriazol-2-yl)-4-methyl-6-[2-methyl-3-[1,3,3,3-tetramethyl-1-[(trimethylsilyl)oxy]disiloxanyl]propyl]-phenol, 3-(4-Methylbenzyliden)campher, 3-Benzylidencampher; 4-(Dimethylamino)-benzoesäure(2-ethylhexyl)ester, 4-(Dimethylamino)benzoesäure-amylester; 4-Methoxybenzalmalonsäuredi(2-ethylhexyl)ester; Dioctylbutylamidotriazon, 4-Methoxyzimtsäure(2-ethylhexyl)ester, 4-Methoxyzimtsäureisopentylester; 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydoxybenzoyl)-benzoesäurehexylester 4-(tert.-Butyl)-4'-methoxydibenzoylmethan, Homomenthylsalicylat, 2-Ethylhexyl-2-hydroxybenzoat, 2-Ethylhexyl-2-cyano-3,3-diphenylacrylat, 3-(4-(2,2-bis Ethoxycarbonylvinyl)-phenoxy)propenyl)-methoxysiloxan / Dimethylsiloxan - Copolymer, Titandioxid, Zinkoxid, enthält.

9. Kosmetische Zubereitung nach einem der vorhergehenden Ansprüche, **dadurch gekennzeichnet, dass** die Zubereitung in Form einer Emulsion vorliegt.
